# EUROPEAN PATENT APPLICATION

(11) **EP 0 578 617 A2**
(43) Date of publication of application: **12.01.1994**
(21) Application number: 93830205.6
(22) Date of filing: 17.05.1993
(51) Int. Cl.: C12N 1/04, C12N 1/14, C12N 11/04, A01N 63/04

(54) **Lyophilized granules containing fungus microorganisms, and a process for the preparation thereof**

(30) Priority: 09.07.1992 IT RM920520
(71) Applicant: SIAPA Società Italo-Americana Prodotti Antiparassitari S.p.A., I-00198 Roma RM (IT)
(72) Inventor: Arteconi, Mario, I-40128 Bologna Bo (IT); Bergonzoni, Paolo, I-40015 S. Vincenzo di Galliera BO (IT); Maini, Paolo, I-40138 Bologna BO (IT); Perrone, Luigi, I-44042 Cento FE (IT); Mallegni, Carlo, I-40015 S. Vincenzo di Galliera BO (IT)
(74) Representative: Di Cerbo, Mario

(57) **Abstract**

Granules of hydrogel for the conservation of fungus microorganisms, characterized in that they comprise essentially the following components, expressed in percentages by weight:
- strains of fungus microorganism 1.0-10
- germinability starter 0.1-50
- humidity 0.5-5.0
- the balance being gel,

and characterized in that they are lyophilized, that they have an apparent density of between 0.10 and 0.50 g/ml, a germinability of the biological principle between 80 and 100%, a conservation period of between 120 and 360 days, and a size of between 0.5 and 2.0 mm.

The invention also extends to a process for preparation of said lyophilized granules.

## Description

The subject of the present invention are lyophilized granules of hydrogel for conserving fungus microorganisms having antagonistic, entomoparasitic and nematocide activities, and a process for the preparation thereof.

It is known that in agriculture there is every increasing interest in the use of living organisms for the control of harmful insects and fungi. These insecticides and fungicides are preferred in order to avoid any damage associated with the use of similar chemical products, such as lack of specificity, residual toxic effects and the fact that the target organisms develop resistance to said products rapidly. The living organisms used under controlled conditions have a specific range of action, and control the target without harming natural predators or useful insects. Examples of this kind of pathogenic or antagonistic agent are: Fusarium, Gliocladium, Trichoderma and the like.

One of the greatest obstacles to use of useful fungi is their conservation period. The biomass obtained by submerged fermentation and conserved in a suspension at 4°C looses its biological activity almost totally after approximately two months. The same biomass, when dried, reduces its activity by one hundred times within a period of four months. Attempts to conserve the biological material in air dried granules of hydrogel have not been altogether satisfactory. In fact, air drying of these granules causes the formation of extremely hard dry granules, which release the biological principle very slowly and with a very low percentage of germinability.

There is therefore, in this specific field, the need to provide a product which is easy to use, sufficiently stable for a period of at least one year, and capable of releasing the biological active principle more rapidly and with an increased percentage of germinability.

Use of lyophilized granules containing fungus microorganisms according to the present invention allows all the positive results mentioned above to be achieved, while at the same time offering other advantages which will be clear from the following.

Subject of the present invention are granules of hydrogel for the conservation of fungus microorganisms, characterized in that they comprise essentially the following components, expressed in percentages by weight:
- strain of fungus microorganism 1.0-10
- germinability starter 0.1-50
- humidity 0.5-5.0
- the balance being gel,

and characterized in that they are lyophilized, that they have an apparent density of between 0.10 and 0.50 g/ml, a germinability of the biological principle between 80 and 100%, a conservation period of between 120 and 360 days, and a size of between 0.5 and 2.0 mm.

The gel is preferably a calcium alginate one.

The strains of fungus microorganism can be selected from the group-comprising Fusarium oxysporum, Trichoderma hartianum, Trichoderma viride, Gliocladium spp., Verticillium lecanii, Metarhizium anisopliae, Beauveria bassiana, Beauveria brongniartii, Hirsutella rhossiliensis, Artrobotrys irregularis and combinations thereof.

The germinability starter can be any organic or inorganic substance that can be used as a source of nitrogen and carbon. It can be selected from the group comprising powdered milk, yeast extract, carbohydrates, vitamins, microelements, aminoacids, protein hydrolyzates, potato homogenizate, by-products deriving from the working of potatoes, beet and milk, cereal flours and combinations thereof.

The granules according to the present invention are easy to use in agriculture, as they can be spread on the land using conventional granule spreaders.

The present invention is not limited to lyophilized granules of hydrogel having a low density and high germinability and conservation time as described above. It also extends to a process for the preparation of said lyophilized granules.

The process for preparation of hydrogel granules containing fungus microorganisms according to the present invention, is characterized essentially by the combination of the following operations:
- solubilization of the gelling agent in water at a concentration lower than 2%;
- addition to the resulting solution of an amount of a strain of fungus microorganism sufficient to obtain a concentration of 10-16 g/l of suspension;
- homogenization of the suspension of gelling agent and biological product;
- optional addition of germinability starter;
- dripping of the homogenized suspension into an aqueous solution under slow stirring containing complexing agent at a concentration of between 0.010 and 0.300 M, maintaining the temperature below 25°C;
- further stirring of the resulting product for a period of time between 10 and 45 minutes;
- separation by filtering of the beads thus formed;
- washing of the beads in water; and
- lyophilization of the beads to obtain lyophilized granules.

In the process according to the present invention, the gelling agent is preferably sodium and/or potassium alginate. The strains of fungus microorganism can be selected from the group comprising Fusarium oxysporum, Trichoderma hartianum, Trichoderma viride, Gliocladium spp., Verticillium lecanii, Metarhizium anisopliae, Beauveria bassiana, Beauveria brongniartii, Hirsutella rhossiliensis, Artrobotrys irregularis and combinations thereof.

The complexing agent can be selected from the group comprising calcium chloride, optionally hydrated, calcium hydroxide, calcium gluconate, calcium nitrate and combinations thereof.

The germinability starter can be any organic or inorganic substance that can be used as a source of nitrogen and carbon. In particular it can be selected from the group comprising powdered milk, yeast extract, carbohydrates, vitamins, microelements, aminoacids, protein hydrolyzates, potato homogenizates, by-products deriving from the working of potatoes, beet, milk, cereal flours and combinations thereof.

Up to now a general description has been given of the subjects of the present invention. With the assistance of the following examples, referring to specific embodiments, a more detailed description of the objects, characteristics, advantages and methods of application will now be given.

### EXAMPLE 1

### Germinability Tests

The tests were carried out by placing, in sterile conditions, 25 lyophilized granules containing as active principle Fusarium oxysporum, in a Petri dish of Potato Dextrose Agar (PDA), repeating the process four times to give a total of 100 granules. As a comparison, air-dried granules containing Fusarium oxysporum were used.

After 48 hours of incubation at 25°C the granules which produced the growth of a well developed colony of Fusarium oxysporum, those which produced no colony, and those which produced colonies of contaminants, either alone or in conjunction with Fusarium oxysporum, were counted.

**Germinability**

| | Lyophilized granules according to invention | Air-dried granules |
|---|---|---|
| Colonies of F. oxysporum | 100% | 60% |
| No colonies | 0 | 38% |
| Colonies of contaminants | 0 | 2% |

### EXAMPLE 2

### Conservation test

The germinability test shown above in Example 1 was repeated at regular intervals of 30 days, examining samples of lyophilized granules containing F. oxysporum, conserved both at room temperature and at 10°C. The data, which are significant, are shown in the following table:

**Germinability**

| | Lyophilized granules according to invention | | Air-dried granules | |
|---|---|---|---|---|
| after 30 days | 100% | 100% | 60% | 60% |
| after 60 days | 100% | 100% | 60% | 60% |
| after 90 days | 100% | 100% | 60% | 60% |
| after 120 days | 100% | 100% | 60% | 60% |
| after 150 days | 100% | 100% | 60% | 60% |
| after 180 days | 100% | 100% | 55% | 60% |
| after 210 days | 100% | 100% | 50% | 60% |
| after 240 days | 99% | 100% | 50% | 60% |
| after 270 days | 99% | 100% | 40% | 50% |
| after 300 days | 98% | 100% | 40% | 50% |
| after 330 days | 97% | 100% | 30% | 50% |
| after 360 days | 95% | 99% | 30% | 40% |

### EXAMPLE 3

The fungus Trichoderma hartianum, obtained by submerged fermentation, is somewhat sensitive to lyophilization on the biomass as it is, and for this reason its drying must be carefully controlled and must take place in the presence of protective agents, such as polysaccharides and the like. The granules obtained by lyophilization of alginate containing T. hartianum gave a more stable product.

The germinability and conservation tests, performed according to examples 1 and 2, gave even for this fungus a germinability of 100% which began to drop off after 180 days conservation, especially in samples conserved at room temperature (80% at 360 days and 90% at 360 days for samples conserved at 10°C).

The comparison granules, air-dried, gave a germinability level of below 50%, with a conservation level which is greatly reduced as early as 120 days.

## Claims

1. Granules of hydrogel for the conservation of fungus microorganisms, characterized in that they comprise essentially the following components, expressed in percentages by weight:
- strains of fungus microorganism 1.0-10
- germinability starter 0.1-50
- humidity 0.5-5.0
- the balance being gel,
and characterized in that they are lyophilized, that they have an apparent density of between 0.10 and 0.50 g/ml, a germinability of the biological principle between 80 and 100%, a conservation period of between 120 and 360 days, and a size of between 0.5 and 2.0 mm.

2. Lyophilized granules of hydrogel for the conservation of fungus microorganisms according to claim 1, in which the gel is a calcium alginate one.

3. Lyophilized granules of hydrogel for the conservation of fungus microorganisms according to claim 1 or 2, in which the strains of fungus microorganism can be selected from the group comprising Fusarium oxysporum, Trichoderma hartianum, Trichoderma viride, Gliocladium spp., Verticillium lecanii, Metarhizium anisopliae, Beauveria bassiana, Beauveria brongniartii, Hirsutella rhossiliensis, Artrobotrys irregularis and combinations thereof.

4. Lyophilized granules of hydrogel for the conservation of fungus microorganisms according to any one of the preceding claims, in which the germinability starter can be selected from the group comprising powdered milk, yeast extract, carbohydrates, vitamins, microelements, aminoacids, protein hydrolyzates, potato homogenizates, by-products deriving from the working of potatoes, beet and milk, cereal flours and combinations thereof.

5. Process for preparation of hydrogel granules containing fungus microorganisms according to the present invention, characterized essentially by the combination of the following operations:
- solubilization of the gelling agent in water at a concentration lower than 2%;
- addition to the resulting solution of an amount of a strain of fungus microorganism sufficient to obtain a concentration of 10-16 g/l of suspension;
- optional addition of germinability starter;
- homogenization of the suspension of gelling agent and biological product;
- dripping of the homogenized suspension into an aqueous solution under slow stirring containing complexing agent at a concentration of between 0.010 and 0.300 M, maintaining the temperature below 25°C;
- further stirring of the resulting product for a period of time between 10 and 45 minutes;
- separation by filtering of the beads thus formed;
- washing of the beads in water; and
- lyophilization of the beads to obtain lyophilized granules.

6. Process for preparation of hydrogel granules containing fungus microorganisms according to claim 5, in which the gelling agent is sodium and/or potassium alginate.

7. Process for preparation of hydrogel granules containing fungus microorganisms according to claim 5 or 6, in which the strains of fungus microorganism are selected from the group comprising Fusarium oxysporum, Trichoderma hartianum, Trichoderma viride, Gliocladium spp., Verticillium lecanii, Metarhizium anisopliae, Beauveria bassiana, Beauveria brongniartii, Hirsutella rhossiliensis, Artrobotrys irregularis and combinations thereof.

8. Process for preparation of hydrogel granules containing fungus microorganisms according to any one of claims 5 to 7, in which the complexing agent is selected from the group comprising calcium chloride, optionally hydrated, calcium hydroxide, calcium gluconate, calcium nitrate and combinations thereof.

9. Process for preparation of hydrogel granules containing fungus microorganism according to any one of claims 5 to 8, in which the germinability starter can be selected from the group comprising powdered milk, yeast extract, carbohydrates, vitamins, microelements, aminoacids, protein hydrolyzates, potato homogenizate, by-products deriving from the working of potatoes, beet and milk, cereal flours and combinations thereof.

10. Hydrogel granules, characterized in that they are obtained in a lyophilized form using the preparation process according to claims 5 to 9.
